(19) Europäisches Patentamt
European Patent Office
Office européen des brevets

(11) Veröffentlichungsnummer: **0 045 024**
**A1**

(12) **EUROPÄISCHE PATENTANMELDUNG**

(21) Anmeldenummer: **81105656.3**

(22) Anmeldetag: **18.07.81**

(51) Int. Cl.³: **C 07 D 211/32**, C 07 D 211/22, C 07 D 401/06

(30) Priorität: **25.07.80 DE 3028198**

(43) Veröffentlichungstag der Anmeldung: **03.02.82 Patentblatt 82/5**

(84) Benannte Vertragsstaaten: **CH DE IT LI NL**

(71) Anmelder: **HOECHST AKTIENGESELLSCHAFT, Postfach 80 03 20, D-6230 Frankfurt/Main 80 (DE)**

(72) Erfinder: **Worm, Manfred, Dr., Erbsenacker 27, D-6200 Wiesbaden (DE)**
Erfinder: **Muth, Karl, Dr., Johann-Strauss-Strasse 33, D-6233 Kelkheim Taunus (DE)**

(54) **Verfahren zur Herstellung von Piperidinyl-alkyl-indolen.**

(57) Die vorliegende Erfindung betrifft ein Verfahren zur Herstellung von Piperidinyl-alkyl-indolen der Formel I

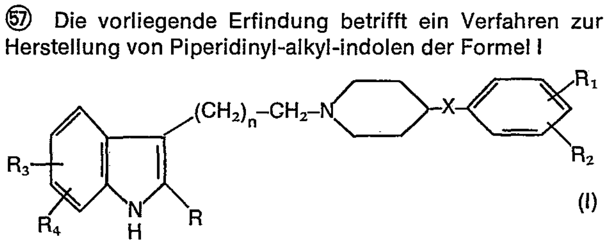

(I)

worin X eine –C=O- oder –CHOH-Gruppe, R Wasserstoff oder Methyl bedeutet, $R_1$ und $R_2$ gleich oder verschieden sind und Wasserstoff, Halogen, geradkettiges oder verzweigtes Alkyl oder Alkoxy mit bis zu 5 C-Atomen, Trifluormethyl, Hydroxy, Phenoxy oder Phenyl bedeuten, $R_3$ und $R_4$ gleich oder verschieden sind und Wasserstoff oder Methoxy bedeuten und n die Zahl 1 oder 2 darstellt, sowie deren pharmazeutisch verträglichen Säureadditionssalze und weiterhin Zwischenprodukte zu deren Herstellung.

EP 0 045 024 A1

0045024

HOECHST AKTIENGESELLSCHAFT HOE 80/F 166 Dr. LA/Fr

Verfahren zur Herstellung von Piperidinyl-alkyl-indolen

Die vorliegende Erfindung betrifft ein Verfahren zur Herstellung von Piperidinyl-alkyl-indolen der Formel I

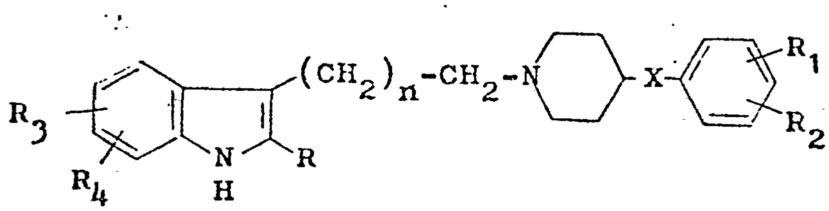

(I)

worin X eine -C=O oder -CHOH-Gruppe, R Wasserstoff oder Methyl bedeuten, $R_1$ und $R_2$ gleich oder verschieden sind und Wasserstoff, Halogen, geradkettiges oder verzweigtes Alkyl oder Alkoxy mit bis zu 5 C-Atomen, Trifluormethyl, Hydroxy, Phenoxy oder Phenyl bedeuten, $R_3$ und $R_4$ gleich oder verschieden sind und Wasserstoff oder Methoxy bedeuten und n die Zahl 1 oder 2 darstellt, sowie deren pharmazeutisch verträglichen Säureadditionssalze, das dadurch gekennzeichnet ist, daß

a) eine Halogenverbindung der Formel II

$$R-\overset{O}{\overset{\|}{C}}-CH_2-(CH_2)_n-CH_2-Hal \qquad (II)$$

worin R und n die zur Formel I genannten Bedeutungen haben und Hal ein Halogenatom bedeutet, mit einem Phenylhydrazin der Formel III

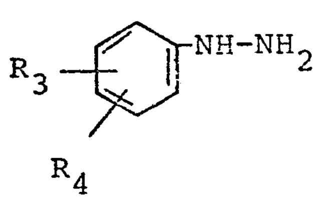

(III)

worin $R_3$ und $R_4$ die zur Formel I genannte Bedeutung haben, umsetzt zu einer Verbindung der Formel IV

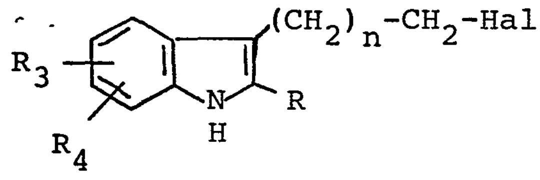

(IV)

und die erhaltene Verbindung der Formel IV gegebenenfalls ohne Isolierung mit einer Verbindung der Formel V

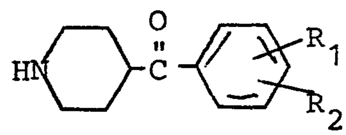

(V)

worin $R_1$ und $R_2$ die zur Formel I genannte Bedeutung haben, zu einer Verbindung der Formel I umgesetzt wird, oder

b) eine Halogenverbindung der Formel II

$$R-\overset{O}{\overset{\|}{C}}-CH_2-(CH_2)_n-CH_2-Hal \qquad \text{(II)}$$

worin R und n die zur Formel I genannte Bedeutung haben und Hal ein Halogenatom bedeutet, zunächst mit einer Verbindung der Formel V

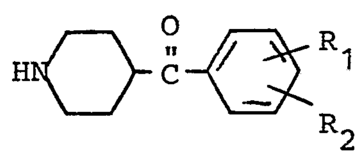

(V)

worin $R_1$ und $R_2$ die zur Formel I genannte Bedeutung haben, umsetzt zu einer Verbindung der Formel VI

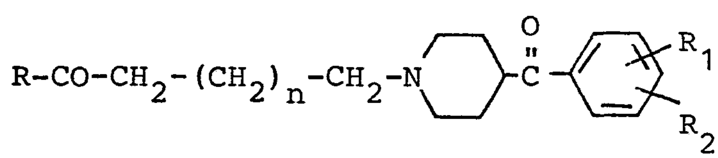

(VI)

und anschließend die erhaltene Verbindung der Formel VI gegebenenfalls ohne Isolierung mit einem Phenylhydrazin der Formel III

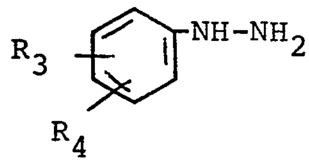

(III)

umsetzt zu einer Verbindung der Formel I, und gegebenenfalls die entweder nach a) oder nach b) erhaltene Verbindung der Formel I, worin X die -C=O-Gruppe darstellt, reduziert zu einer Verbindung der Formel I, worin X die -CHOH-Gruppe bedeutet.

Hal in der Formel II bedeutet vorzugsweise Chlor, Brom oder Jod, insbesondere Chlor.

Piperidinyl-alkyl-indole der Formel I sowie Verfahren zu deren Herstellung sind bereits in der DE-OS 27 08 913 beschrieben.

Nach dem ersten der dort angegebenen Verfahren wird eine Verbindung der Formel VII

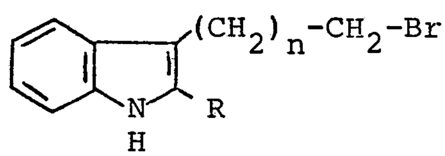

(VII)

mit einem 4-Benzoylpiperidin der Formel V

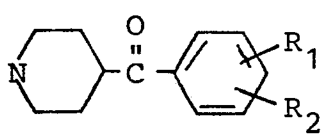

(V)

umgesetzt zu einer Verbindung der Formel I.
Die Herstellung der 3-(ω-Brom-alkyl)-indole der Formel VII erfolgt jedoch nach dem folgenden mehrstufigen Schema in literaturbekannter Weise (siehe z.B.: T.Hoshino et al., Justus Liebigs Ann.d.Chem. 520, 19 (1935); J.J. Grandberg, Zh. Obsheh.Khim. 27, 3342 (1957), C.A. 52, 9071g (1958); R.A. Robinson, U.S. Patent 2 908 691 (1959), C.A. 56, 3455 (1962); T.Vitali, Bol. Sci.Fac.Chim. Ind. Bologna 17, 84

(1959), C.A. 54, 19644a (1960); F. Lingens et al., Justus Liebigs Ann.Chem. 662, 139 (1963)):

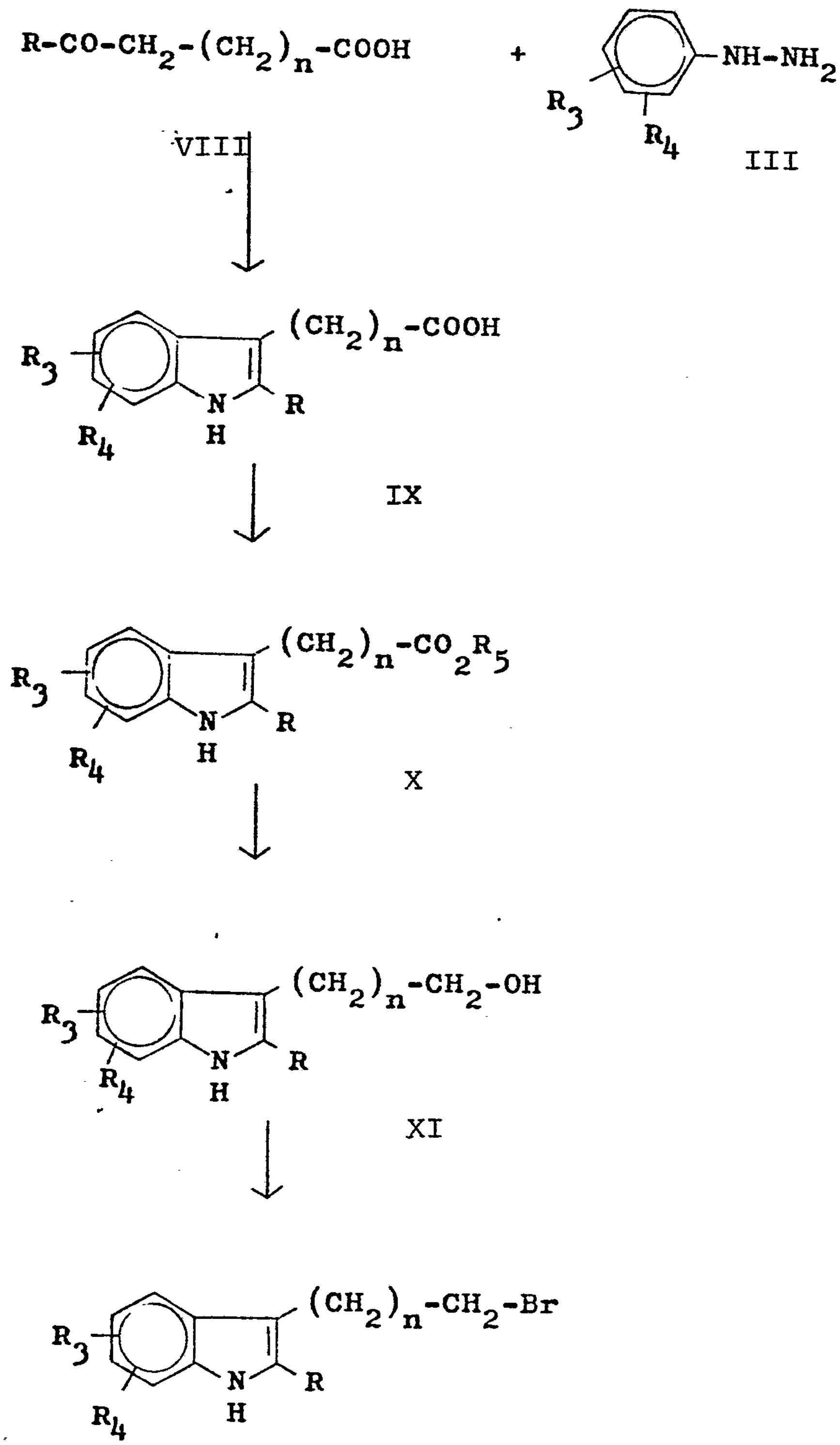

VIII III IX X XI XII

wobei R, $R_3$, $R_4$ und n die zur Formel I angegebene Bedeutung haben und $R_5$ für Methyl oder Äthyl steht.

Nach dem zweiten in der DE-OS 27 08 913 angegebenen Verfahren läßt man ein Indol der Formel XIII

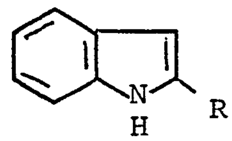

(XIII)

mit Oxalylchlorid regaieren und setzt das Reaktionsprodukt mit einem Benzoylpiperidin der Formel V

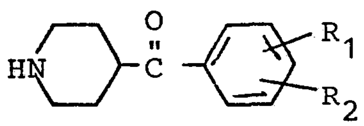

(V)

um zu einer Verbindung der Formel XIV

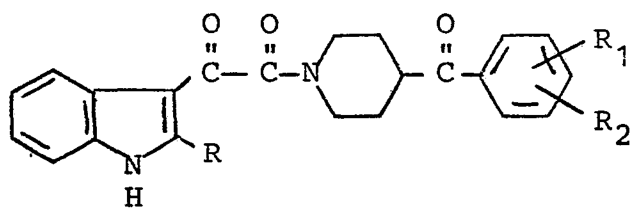

(XIV)

deren Reduktion jedoch nur zu einer Verbindung der Formel I führt, worin X = CHOH und n die Zahl 1 bedeutet.

Demgegenüber führt das Verfahren der vorliegenden Erfindung gemäß den Varianten a) und b) von leicht zugänglichen literaturbekannten Ausgangsstoffen in einer jeweils 2-stufigen Synthese zu Verbindungen der Formel I, wobei die Zwischenprodukte der Formel IV bzw. VI nicht isoliert zu werden brauchen. Die Vorteile des erfindungsgemäßen Verfahrens gegenüber dem Stand der Technik liegen daher in der Einsparung von drei Stufen, wobei insbesondere der Wegfall der Reduktion des Esters der Formel X zum Alkohol der Formel XI, die mit Lithiumaluminiumhydrid oder ähnlichen Reduktionsmitteln durchgeführt wird und technisch hinsichtlich der Sicherheitsvorkehrungen erhebliche Aufwendungen verursacht, ins Gewicht fällt.

Die Reaktion gemäß der ersten Stufe der Variante a) des erfindungsgemäßen Verfahrens ist überraschend, da erwartet

werden konnte, daß das Halogen durch den Stickstoff des Phenylhydrazins substituiert werden würde. Ferner ist aus der Literatur (s.z.B. J.J. Grandberg et al., Khim.Geterotsikl. Soedin, 1970, 1495, Chem. Inform. 1971, 15 - 282; J.J. Grandberg, Khim.Geterotsikl. Soedin. 1974, 579, Chem.Inform. 1974, 35 - 244; J.J. Grandberg, Dokl.Akad.Nauk. SSSR 176, 583 (1967), C.A. 68, 104883) bekannt, daß aus Phenylhydrazin und einer Verbindung der Formel II (z.B. mit Hal= Chlor, R= H oder $CH_3$ und n = 1 oder 2) unter ähnlichen Reaktionsbedingungen Aminoalkylindole der Formel XV

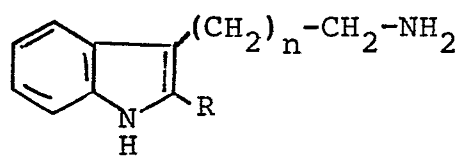

(XV)

anstatt Verbindungen der Formel IV entstehen.

Hinsichtlich der 2. Reaktionsstufe der erfindungsgemäßen Verfahrensvariante b) ist als überraschend anzusehen, daß ein N-Alkyl-4-benzoylpiperidin der Formel VI direkt mit einem Phenylhydrazin der Formel III umgesetzt werden kann, ohne daß beide Carbonylgruppen der Verbindung der Formel VI mit dem Phenylhydrazin reagieren.

Die erfindungsgemäßen Verfahrensvarianten werden wie folgt durchgeführt:

a) Die Halogenverbindung der Formel II wird mit dem Phenylhydrazin der Formel III in einem inerten Lösungsmittel unter sauren Bedingungen und bei erhöhter Temperatur zur Reaktion gebracht. Als Säure können Mineralsäuren, wie z.B. Salz- oder Schwefelsäure, oder Lewissäuren wie z.B. $AlCl_3$ oder $ZnCl_2$ verwendet werden.

Als inerte Lösungsmittel kommen aliphatische Alkohole, z.B. $C_{1-6}$-Alkanole, bevorzugt Methanol oder Äthanol, ferner aromatische Kohlenwasserstoffe, z.B. Toluol, oder

Eisessig infrage.

Die Reaktion wird bei Temperaturen von Raumtemperatur bis 150°C, bevorzugt bei 40 bis 100°C durchgeführt. Das dabei erhaltene Halogenindol der Formel IV kann z.B. durch Kristallisation oder Destillation gereinigt werden. Die bevorzugte Darstellungsform besteht jedoch darin, daß man es ohne Isolierung und Reinigung unmittelbar mit dem 4-Benzoylpiperidin der Formel V umsetzt. Diese Umsetzung erfolgt vorzugsweise in einem inerten Lösungsmittel, wie z.B. Dimethylformamid, $C_{1-6}$-Alkanole, insbesondere Methanol, Äthanol, Isopropanol oder n-Butanol, Ketone wie z.B. Butanon, in Gegenwart oder Abwesenheit einer Base wie z.B. anorganische Basen, insbesondere Kaliumcarbonat, in Gegenwart oder Abwesenheit eines Katalysators, wie z.B. Kaliumjodid, und bei einer Temperatur von Raumtemperatur bis 150°C, wobei die Umsetzung bei der Siedetemperatur des Lösungsmittels bevorzugt ist.

b) Die Umsetzung der Halogenverbindung der Formel II mit dem 4-Benzoylpiperidin der Formel V erfolgt vorzugsweise in einem inerten Lösungsmittel wie $C_{1-6}$-Alkanole, insbesondere Metahnol oder Äthanol, oder Ketone wie z.B. Butanon oder Dimethylformamid oder ohne Lösungsmittel, wobei die Halogenverbindung der Formel II im Überschuß eingesetzt und nach der Reaktion wieder rückgewonnen werden kann. Die Reaktion erfolgt auch hier mit oder ohne Base wie z.B. anorganische Basen, insbesondere Kaliumcarbonat, mit oder ohne Katalysator wie z.B. Kaliumjodid und bei Temperaturen zwischen Raumtemperatur und 150°C, vorzugsweise bei 50 bis 100°C. Das dabei erhaltene N-Alkyl-4-benzoylpiperidin der Formel VI läßt man durch Zutropfen von Phenylhydrazin der Formel III weiter reagieren, wobei die Temperatur tief, d.h. zwischen -20 und +20°C, vorzugsweise bei -10 bis 0°C gehalten wird. Die Temperatur wird danach langsam, vorzugsweise innerhalb von 1 bis 5 Stunden auf 20 bis 30°C erhöht, der saure Katalysator, vorzugsweise Salz- oder Schwefelsäure hinzugefügt und noch weiter , bevorzugt bei erhöhter Temperatur, z.B. bei 50 bis 100°C ausreagieren lassen. Diese Reaktion wird vorzugsweise wieder in einem inerten Lösungsmittel wie z.B. einem $C_{1-6}$-Alkanol, insbesondere Methanol, Äthanol oder in Eisessig durchgeführt.

Die für das erfindungsgemäße Verfahren benutzten Ausgangsverbindungen sind literaturbekannt und können leicht hergestellt werden. Die Herstellung der Halogenverbindungen der Formel II ist z.B. beschrieben in C.A. 68, 104883; Org. Synth. 31, 74 (1951); U.S. Patent 2 370 392; P. Kipping, J. Chem. Soc. 55, 332; Ishimura, J.Chem.Soc.Jap. Pure Chem. Sect. 76, 1253 (1955); C.A. 1957, 17728).

Die Synthesemöglichkeiten für die 4-Benzoylpiperidine der Formel V ist ausführlich beschrieben in DE-OS 27 08 913.

Die Verbindungen der Formel I können als Arzneimittel mit sedierender, blutdrucksenkender und analgetischer Wirkung verwendet werden.

Beispiel 1:

2-Methyl-3-(3-chlor-n-propyl)-indol (IV)

In eine $70^{O}$ warme Lösung von 33,6 g 1-Chlor-hexanon-(5) in 50 ml Eisessig wurden während 30 Min. 38,7 g Phenylhydrazinhydrochlorid eingetragen. Es wurde 1 Std. bei $70^{O}$ und 30 Min. bei $100^{O}$ nachgerührt, auf $5^{O}$ abgekühlt, filtriert und das Filtrat im Vakuum eingeengt. Der Rückstand wurde in 100 ml Methylenchlorid gelöst und anschließend zweimal mit je 125 ml 2 n Natronlauge, einmal mit je 100 ml 2n Salzsäure und zweimal mit je 150 ml Wasser gewaschen. Nach dem Trocknen ($Na_2SO_4$) und Abdestillieren des Lösungsmittels im Vakuum wurden 50,8 g 2-Methyl-3-(3-chlor-n-propyl)-indol (95% d.Th) als Öl erhalten. Das Produkt ist gaschromatographisch 96,9% rein und kann ohne Reinigung weiter umgesetzt werden. Man kann das ölige Produkt aber auch destillieren (Sdp. ca. $150^{O}$/0,1 mm).

Beispiel 2:

2-Methyl-3-(2-chloräthyl)-indol (IV)

Analog dem Beispiel 1 erhält man aus 20,0 g 1-Chlor-pentanon-(4) und 25,6 g Phenylhydrazinhydrochlorid 23,5 g der Titelverbindung.

Beispiel 3:

2-Methyl-3-{3-[4-(4-fluorbenzoyl)-piperidin-1-yl]-propyl}-indol (I)

a) Eine Mischung aus 25,0 g 4-(4-Fluor-benzoyl)-piperidin-hydrochlorid- 26,0 g 2-Methyl-3-(3-chlor-n-propyl)-indol, 54,2 g Kaliumcarbonat, 0,2 g Kaliumjodid und 400 ml Äthanol wurde

7 Stunden unter Rückfluß gekocht, auf 25° gekühlt und filtriert. Das Filtrat wurde mit 400 ml Wasser verdünnt, wobei 23,7 g der Titelverbindung auskristallisierten, Fp. 135 - 137°.

b) Zu einer kalten (-10°) Lösung von 10,0 g 1-(5-Oxo-n-hexyl)-4-(4-fluor-benzoyl)-piperidin-hydrochlorid in 30 ml Methanol wurden während 45 Min. 3,16 g Phenylhydrazin hinzugefügt. Nach dem Rühren (1 Std. bei - 10° bis + 10°) wurden 10,5 ml konz. Schwefelsäure während 10 Min. zugetropft, die Mischung auf 70° bis 75° erwärmt und bei dieser Temperatur 1 1/2 Std. gehalten. Nach dem Abkühlen auf 20° wird die Mischung zu 100 g Eis, 20 ml Natronlauge (48 %-ig) und 100 ml Methylenchlorid gegeben, die organische Phase abgetrennt,die wäßrige Phase nochmals mit 50 ml Methylenchlorid extrahiert, die vereinigten organischen Phasen mit Wasser gewaschen, über Natriumsulfat getrocknet und im Vakuum zu einem Öl konzentriert.
Ausbeute: 15 g der Titelverbindung.
Fp. 131 - 134° nach Kristallisation aus Isopropanol.

Beispiel 4:

1-(5-Oxo-n-hexyl)-4-(4-fluorbenzoyl)-piperidin-hydrochlorid (VI )

Eine Mischung aus 6,8 g 4-(4-Fluorbenzoyl)-piperidin-hydrochlorid, 4,2 g 1-Chlor-hexanon-(5), 4,6 g Kaliumcarbonat und 20 ml DMF wurde 3 1/2 Std. unter Rückfluuß gekocht, auf 25° gekühlt, mit 100 ml Wasser verdünnt und 2 x mit je 75 ml Methylenchlorid extrahiert. Die organische Phase wurde mit Wasser gewaschen, und zu einem Öl im * HCl/Diisopropyläther zum kristallinen Hydrochlorid umgewandelt wurde.
Ausbeute: 7,1 g Fp. nach Umkristallisation: 179 - 181°.

* Vakuum konzentriert, welches mit

Beispiel 5:

1-(4-Oxo-n-pentyl)-4-(4-fluorbenzoyl)-piperidin-hydrochlorid (VI )

Analog dem Beispiel 4 erhält man aus 20,7 g 4-(4-Fluorbenzoyl)-piperidin-hydrochlorid und 43,5 g 1-Chlor-pentanon-(4) 21,0 g der Titelverbindung vom Fp. 186 - 188° nach Umkristallisation aus Methanol/Diisopropyläther.

Beispiel 6:

1-(4-Oxo-n-pentyl)-4-(4-fluorbenzoyl)-piperidin-hydrobromid (VI )

Diese Verbindung erhält man analog * Hydrochlorid von Beispiel 5; sie schmilzt bei 160 - 162°.

* dem

Beispiel 7:

2-Methyl-3-{2-[4(4-fluorbenzoyl)-piperidin-1-yl]-äthyl}-indol (I)

a) Analog dem Beispiel 3a erhält man aus 22,6 g 4-(4-Fluorbenzoyl)-piperidin-hydrochlorid und 18 g 2-Methyl-3-(2-chloräthyl)-indol 36,3 g der Titelverbindung.

b) Analog dem Beispiel 3 b erhält man aus 14 g 1-(4-Oxo-n-pentyl)-4-(4-fluorbenzoyl)-piperidin-hydrochlorid und 4,6 g Phenylhydrazin 17,8 g der Titelverbindung.

0045024

c) Analog dem Beispiel 3 b erhält man aus 15 g 1-(4-Oxo-n-pentyl)-4-(4-fluorbenzoyl)-piperidin-hydrobromid und 4,6 g Phenylhydrazin 16,3 g der Titelverbindung.

Patentansprüche:

1. Verfahren zur Herstellung von Piperidinyl-alkyl-indolen der Formel I

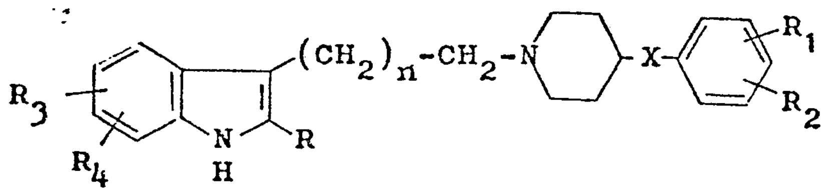

worin X eine -C=O oder -CHOH-Gruppe, R Wasserstoff oder Methyl bedeuten, $R_1$ und $R_2$ gleich oder verschieden sind und Wasserstoff, Halogen, geradkettiges oder verzweigtes Alkyl oder Alkoxy mit bis zu 5 C-Atomen, Trifluormethyl, Hydroxy, Phenoxy oder Phenyl bedeuten, $R_3$ und $R_4$ gleich oder verschieden sind und Wasserstoff oder Methoxy bedeuten und n die Zahl 1 oder 2 darstellt, sowie deren pharmazeutisch verträglichen Säureadditionssalze, dadurch gekennzeichnet, daß

a) eine Halogenverbindung der Formel II

$$R-\overset{O}{\overset{\parallel}{C}}-CH_2-(CH_2)_n-CH_2-Hal$$

worin R und n die zur Formel I genannten Bedeutungen haben und Hal ein Halogenatom bedeutet, mit einem Phenylhydrazin der Formel III

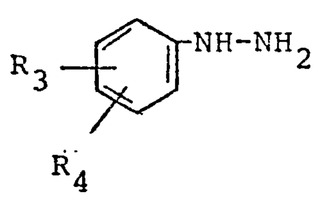

worin $R_3$ und $R_4$ die zur Formel I genannte Bedeutung haben, umsetzt zu einer Verbindung der Formel IV

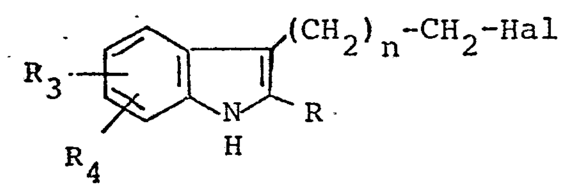

(IV)

und die erhaltene Verbindung der Formel IV gegebenenfalls ohne Isolierung mit einer Verbindung der Formel V

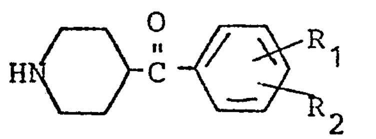

(V)

worin $R_1$ und $R_2$ die zur Formel I genannte Bedeutung haben, zu einer Verbindung der Formel I umgesetzt wird, oder

b) eine Halogenverbindung der Formel II

$$R-\overset{O}{\overset{\|}{C}}-CH_2-(CH_2)_n-CH_2-Hal \qquad (II)$$

worin R und n die zur Formel I genannte Bedeutung haben und Hal ein Halogenatom bedeutet, zunächst mit einer Verbindung der Formel V

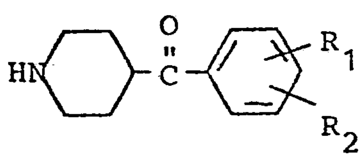

(V)

worin $R_1$ und $R_2$ die zur Formel I genannte Bedeutung haben, umsetzt zu einer Verbindung der Formel VI

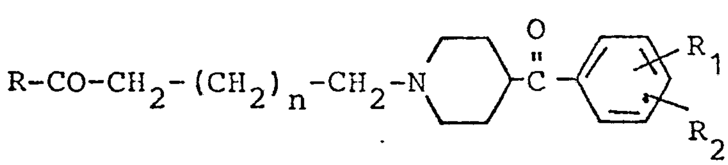

(VI)

und anschließend die erhaltene Verbindung der Formel VI gegebenenfalls ohne Isolierung mit einem Phenylhydrazin der Formel III

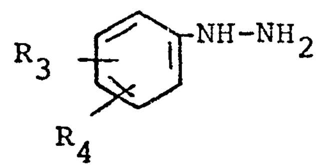

umsetzt zu einer Verbindung der Formel I, und gegebenenfalls die entweder nach a) oder nach b) erhaltene Verbindung der Formel I, worin X die -C=O-Gruppe darstellt, reduziert zu einer Verbindung der Formel I, worin X die -CHOH-Gruppe bedeutet und ggfs. in üblicher Weise Säureadditionssalze herstellt.

2. Verfahren nach Anspruch 1, dadurch gekennzeichnet, daß man eine Halogenverbindung der Formel II

$$R-\overset{O}{\overset{\|}{C}}-CH_2-(CH_2)_n-CH_2-Hal$$

worin R und n die zur Formel I genannten Bedeutungen haben und Hal ein Halogenatom bedeutet, unter sauren Bedingungen und bei erhöhter Temperatur mit einem Phenylhydrazin der Formel III

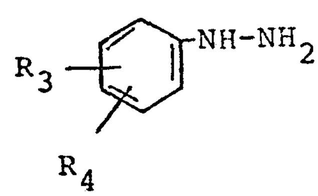

worin $R_3$ und $R_4$ die zur Formel I genannte Bedeutung haben, zu einer Verbindung der Formel IV

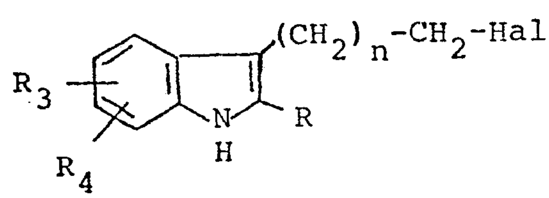

umsetzt.

3. Verfahren nach Anspruch 2, dadurch gekennzeichnet, daß die Reaktion in Gegenwart einer Mineralsäure und bei einer Temperatur von 40 bis 100°C durchgeführt wird.

4. Verfahren nach Anspruch 1, dadurch gekennzeichnet, daß eine Verbindung der Formel VI

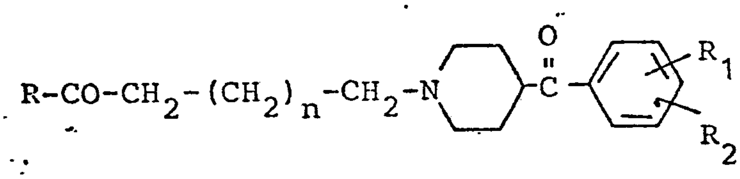

worin R, $R_1$, $R_2$ und n die zur Formel I genannten Bedeutungen haben, mit einem Phenylhydrazin der Formel III

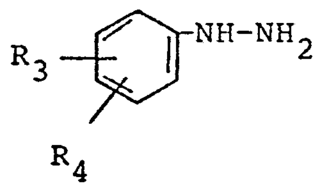

worin $R_3$ und $R_4$ die zur Formel I genannten Bedeutungen haben, umsetzt.

5. Verfahren nach Anspruch 4, dadurch gekennzeichnet, daß man zu einer Verbindung der Formel VI bei einer Temperatur von -20 bis +20°C das Phenylhydrazin zutropfen läßt, die Temperatur innerhalb von etwa 1 bis 5 Stunden auf bis etwa 20 bis 30°C erhöht und nach Zugabe einer Mineralsäure bei etwa 50 bis 100°C ausreagieren läßt.

6. Verbindung der Formel VI

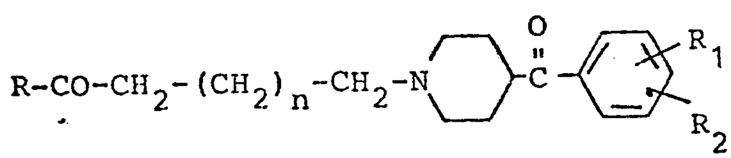

worin R Wasserstoff oder Methyl bedeutet, $R_1$ und $R_2$ gleich oder verschieden sind und Wasserstoff, Halogen, geradkettiges oder verzweigtes Alkyl oder Alkoxy mit bis zu 5-C-Atomen, Trifluormethyl, Hydroxy, Phenoxy oder Phenyl und n die Zahl 1 oder 2 bedeuten.

7. Verfahren zur Herstellung einer Verbindung der Formel VI

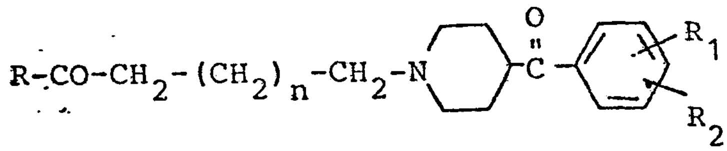

worin R, $R_1$, $R_2$ und n die in Anspruch 6 genannten Bedeutungen haben, dadurch gekennzeichnet, daß man eine Halogenverbindung der Formel II

$$R-\overset{O}{\overset{\|}{C}}-CH_2-(CH_2)_n-CH_2-Hal$$

worin R und n die in Anspruch 6 genannten Bedeutungen haben und Hal ein Halogenatom bedeutet, mit einer Verbindung der Formel V

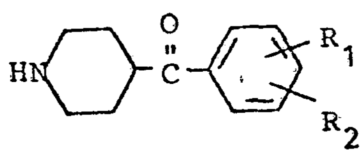

worin $R_1$ und $R_2$ die oben genannten Bedeutungen haben, umsetzt.

Europäisches Patentamt

# EUROPÄISCHER RECHERCHENBERICHT

0045024

Nummer der Anmeldung

EP 81 10 5656

| Kategorie | EINSCHLÄGIGE DOKUMENTE<br>Kennzeichnung des Dokuments mit Angabe, soweit erforderlich, der maßgeblichen Teile | betrifft Anspruch | KLASSIFIKATION DER ANMELDUNG (Int. Cl.³) |
|---|---|---|---|
| D,X | CHEMICAL ABSTRACTS, Band 68, Seite 10118, Zusammenfassung 104883k<br>COLUMBUS, OHIO (US)<br>& Dokl.Akad.Nauk SSSR 176(3) 583-585(1967)<br>I.I.GRANDBERG u.a.: "New method of synthesizing important biogenic amines"<br>* ganze Artikel *<br>-- | 1-5 | C 07 D 211/32<br>/22<br>C 07 D 401/06 |
| D | DE - A - 2 708 913 (HOECHST AG)<br>* Patentansprüche *<br>-- | 1-5,7 | RECHERCHIERTE SACHGEBIETE (Int. Cl.³)<br>C 07 D 211/18<br>/20<br>/22<br>/32<br>C 07 D 209/14<br>C 07 D 401/06 |
| A | FR - A - 2 331 557 (A.H.ROBINS)<br>* ganze Artikel *<br>-- | 6 | |
| D | US - A - 2 908 691 (R.A.ROBINSON)<br>* Seite 2, Spalte 1, Zeilen 25-40 *<br>---------- | 1-5 | |

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Den Haag | 11-09-1981 | MAISONNEUVE |